# EUROPEAN PATENT SPECIFICATION

(11) **EP 1 626 653 B1**
(45) Date of publication and mention of the grant of the patent: **19.09.2007**
(21) Application number: 04750467.5
(22) Date of filing: 22.04.2004
(51) Int. Cl.: A61B 5/00, A61K 8/18

(54) **PAD FOR CLEANING AND HYDRATING SKIN**
KISSEN ZUR HAUTREINIGUNG UND HAUTBEFEUCHTUNG
DISQUE POUR NETTOYER ET HYDRATER LA PEAU

(30) Priority: 22.04.2003 US 464691 P
(43) Date of publication of application: 22.02.2006
(73) Proprietor: Biodel, Inc., Danbury, CT 06810-6352 (US)
(72) Inventor: STEINER, Solomon, S., Mount Kisco, NY 10549 (US)
(74) Representative: HOFFMANN EITLE
(86) International application number: PCT/US2004/012413
(87) International publication number: WO 2004/093833

(56) References cited:
- WO-A-00/65143
- US-A- 3 998 215
- US-A- 4 515 162

## Description

### FIELD OF THE INVENTION

The present invention is directed at devices for cleaning and hydrating the surface of the skin and more specifically for devices that can be used to enhance performance of transdermal devices.

### BACKGROUND OF THE INVENTION

Various devices, such as non-invasive blood glucose monitors, cardiac function monitors, electro-myograph monitors, and wrist watches must be worn on the skin. These devices are not able to obtain an accurate measurement for a number of reasons. First, the condition of the skin when the device is first applied is variable from occasion to occasion for the same patient and from patient to patient. Second, as the skin loses moisture over time, the performance of these devices and their reliability can be adversely affected. Third, some individuals develop a hypersensitivity or rash when the material of the device, (usually metal) is in contact with the skin for prolonged periods of time.

Therefore, it is an object of the invention to provide a device that cleans and hydrates the skin.

It is a further object of the invention to provide the use of a device for cleansing and hydrating an area on the surface of a person's skin for applying a delivery, sensing or monitoring device to this area.

U.S. patent number 4,515,162 relates to an electropad.

U.S. patent number 3,998,215 refers to biomedical electrode conductive gel pads.

### BRIEF SUMMARY OF THE INVENTION

A pad for improving the accuracy of analytical or delivery devices that are applied to the skin is described herein. The pad is a two-sided pad, where the first side contains a cleansing formulation and the second side contains a hydrating formulation. The pad has an inert, liquid impervious backing separating the first and second sides. The material or device is applied to the skin to cleanse and hydrate the skin before a monitoring device is applied to the skin, thereby reducing the electrical impedance of the skin and the skin remains hydrated during the time period that the monitoring device is in contact with the skin. The pad produces beneficial results with devices that operate at both high and low frequencies, by stabilizing the signal that is measured.

### BRIEF DESCRIPTION OF THE DRAWINGS

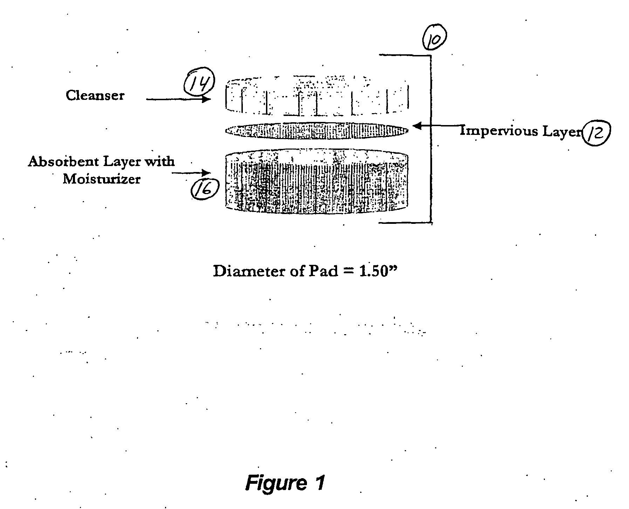

Figure 1 is a schematic of the pad device.

### DETAILED DESCRIPTION OF THE INVENTION

More accurate measurements can be obtained if (1) the skin has been cleansed of dirt and dead flaking skin to a uniform degree such that the electrical impedance of the skin is reduced; (2) the skin is sufficiently hydrated; (3) the skin retains the majority of its hydrated state over the time period that the device is worn; (4) the electrical properties of the contact remain relatively constant over time despite movement; and (5) the skin is treated in a manner to reduce the probability and severity of developing dermatitis, hypersensitivity reactions, rashes, and irritations.

### 1. Pad

A pad for improving the performance of transdermal devices has been developed. As shown in Figure 1, the pad consists of a two-sided pad 10, where the first side contains a cleansing formulation 14 and the second side contains a hydrating formulation 16. The pad is backed with a stable and an impervious material 12, such as a TEFLON® (E. I. du Pont de Nemours and Co) sheet or plasticized paper. Alternative backing materials include polymers, such as polyesters such as MYLAR^{®} (E.I. du Pont de Nemours and Co.), polyamides such as nylon, and polyolefins such as polypropylene, polyethylene, and polyvinyl chloride.

### A. Cleansing Surface

One surface of the pad is made of a mildly abrasive material, such as gauze or a woven mesh of any elastic fiber or metal, such as stainless steel. The first side is impregnated with a cleansing formulation which is a solution or suspension containing an alcohol (such as ethyl or isopropyl alcohol), water, a preservative, or a fragrance, (such as benzaldehyde, which is artificial almond scent), or a combination thereof. This side cleanses the skin of dirt and removes dead flaking skin to a uniform degree, such that the electrical impedance of the skin is reduced below 10,000 ohms/cm². In the preferred embodiment the electrical impedance of the skin is reduced to below 5,000 ohms/cm².

### B. Hydrating Surface

The other surface can be made with the same material as the first side or of a different material from the first side, such as a foam pad. The second side is impregnated with a solution, emulsion or suspension containing a cream, hydrogel, lotion, oil, or moisturizer, or combination thereof. Other substances known to one skilled in the art, including bioactive substances, and substances which alter penetration (such as chemical penetration enhancers) may also be included in the solution, emulsion or suspension. Optionally, the second side also includes a fragrance. In the preferred embodiment, urea is added to the solution, emulsion or suspension to retain moisture in the skin, ensuring that the skin is sufficiently hydrated over the time period that the device is worn. The solution, emulsion or suspension may also contain ions to act as an electrolyte and by so doing ensure that the electrical properties of the contact remain relatively constant over the time the device is worn, despite movement. The ingredients in the solution, emulsion or suspension are selected to reduce the probability and severity of developing dermatitis, hypersensitivity reactions, rashes, and skin irritations. A sufficient amount of the solution, emulsion, or suspension is added to the pad to coat the skin so that the solution, emulsion, or suspension is absorbed by the epidermis.

### 2. Method of using the Pad

The pad is used to enhance the effectiveness of any of a variety of different devices. Representative devices include ultrasound delivery devices, analyte measuring devices, and drug delivery devices. The pad may be combined with a monitoring device to obtain many different measurements, including galvanic skin response (GSR), pulse propagation velocity, vector or impedance cardiography, electrocardiogram (EKG), electroencephalogram (BEG), iontophoretic drug delivery, and reverse iontophoresis for measuring interstitial fluids. Monitoring devices include blood glucose monitors, cardiac function monitors, electromyography monitors, and wrist watches.

The pad is applied to an area of the skin to cleanse and hydrate the skin. Then, the device is applied to the same area of the skin. The skin should remain hydrated throughout the time period that the monitoring device is in contact with the skin. By cleansing the skin before applying the measuring device, the electrical impedance of the skin is reduced below 10,000 ohms/cm². In the preferred embodiment the electrical impedance of the skin is reduced to below 5,000 ohms/cm². The measuring device may be operated at a wide range of frequencies. The frequency may range from subsonic (0.1 Hz) to 200 MHz. In the measurement of impedance at frequencies in the MHz range, the major consideration in performing accurate measurements is the reduction over time of the variability of the impedance itself and of the resonant frequency.

The pad is applied for a sufficient time to wet the skin so that the variability of the impedance and the resonant frequency are reduced compared to the variability present when a pad is not used with the same device.

The compositions and methods described herein will be further understood by reference to the following non-limiting examples.

### Examples

### Example 1: Effects of Pad use on Impedance at low frequency (60 Hz)

Six healthy volunteers between the ages of 19 and 65 served as subjects in this study. Two of the volunteers were females and four were males. The subjects were divided into two equal sized groups, each containing one female and two males.

Impedance measurements were made at a frequency of 60 Hz through two circular silver-silver chloride (Ag-AgCl) electrodes placed approximately 8 cm apart on the forehead of the volunteer. The electrodes were held in place with adhesive tape.

The pad contained 30% isopropyl alcohol on its gauze side and a hydrogel containing 2% of Sea Buckthorn Oil, Purified Water, Glucono Delta Lactone, Glycerin, Hydroxyethylcellulose, Sodium Hydroxide, and Chlorhexidine Gluconate, and Methylparabene, as preservatives, on its foam side.

Group 1 had the impedance of their forehead measured twice on the same day, ten minutes apart without the use of the pad. (Experimental day 1)

This procedure was repeated at least one day and not more than six days later (Experimental day 2). Five minutes prior to the second impedance measurement, the subject's forehead was rubbed with the gauze side of the pad and then daubed with the foam side of the pad.

Group 2 had the pad applied on the Experimental day 1, but not on the second experimental day. This procedure serves as a control for the effects, if any, of two consecutive impedance measurements made within 10 minutes on the same day.

### Results

Impedance readings made without the application of the pad were uniformly high and variable, with an average impedance of 26,000 ohms and a range from 18,000 to 38,000 ohms. By contrast, impedance readings made within ten minutes of the application of the pad were considerably lower, with an average impedance of 4,800 ohms and a range from 4,300 to 5,200 ohms.

### Example 2: Effects of pad use on Impedance at high Frequency (20 to 100 MHz)

### Method

Normal healthy volunteers wore two Pendragon Medical glucose sensing devices, (Pendra®), one on each wrist for periods in excess of three hours. The Pendra® measures blood glucose by impedance spectroscopy, sweeping across frequencies from 20 to 100 MHz.

The pad was applied as in Example 1 to one of the two wrists and not to the other wrist. The pad contained 30% isopropyl alcohol on its gauze side and a formulation containing 2% of Sea Buckthorn Oil from Alpha Engineering GmbH, (Sanddorn-Fruchtfleisch-Rohol, Type E Charge:241001) and additional materials listed below, on its foam side. Comparisons were made between the treated and un-treated wrist as well as between the formulation applied to the foam portion of the pad. Formulation A was a hydrogel containing Purified Water, Glucono Delta Lactone, Glycerin, Hydroxyethylcellulose, Sodium Hydroxide, and Chlorhexidine Gluconate and Methylparabene, as preservatives.

Formulation B was the same hydrogel as Formulation A, with the addition of a small amount of a fragrance, composed of one-third ethyl alcohol; and two-thirds water and Benzaldehyde.

Formulation C was a hypo-allergenic cream lotion containing Purified Water, Mineral Oil, Petrolatum, Sorbitol, Stearic Acid, Lanolin, Lanolin Alcohol, Cetyl Alcohol, Glyceral Sterate/PEG-1.00Sterate, Triethanolamine, Dimethicone, Propylene Glycol, Microcrystalline Wax, Tri (PPG-3 Myristyl Ether) Citrate, Disodium EDTA, Xanthan Gum and Methylparabene, Ethylparabene, and Propylparabene, Butylparabene and Methyldibromo Glutaronitrile, as preservatives.

Formulation D was the same hypo-allergenic cream lotion as Formulation C, with the addition of a small amount of a fragrance, composed of one-third ethyl alcohol and two-thirds water and Benzaldehyde.

Formulation E was a hypo-allergenic oil gel containing Mineral Oil, Hydrogenated Butylene/Ethylene/Styrene Copolymer, Hydrogenated Ethylene/Styrene Copolymer, Tocopheryl Acetate, and Aloe Baradensis Extract.

Formulation F was the same hypo-allergenic oil gel as Formulation E, with the addition of a small amount of a fragrance, composed of one-third ethyl alcohol and two-thirds water and Benzaldehyde.

### Results

Pad treatment reliably reduced, over time, the variability of the impedance itself and the reduction over time of the variability of the resonant frequency for most of the formulations. Formulations A, B, C, and D all stabilized the impedance measurement and the resonant frequency. Formulations E and F produced similar results to those obtained when no pad was used.

## Claims

1. A two-sided pad (10) comprising
a first side of the pad (14) comprising a cleansing formulation, a second side of the pad (16) comprising a hydrating formulation, and an inert, liquid impervious backing (12) separating the first and second sides.

2. The two-sided pad (10) of claim 1, wherein the first side (14) comprises a mildly abrasive material.

3. The two-sided pad (10) of claim 2, wherein the second side (16) comprises the same material as the first side.

4. The two-sided pad (10) of claim 1, wherein the cleansing formulation is a solution or suspension comprising an alcohol, water, a preservative, or a fragrance, or a combination thereof.

5. The two-sided pad (10) of claim 1, wherein the hydrating formulation is a solution, emulsion or suspension, comprising a hydrogel, lotion, oil, moisturizer, urea or combination thereof.

6. The two-sided pad according to any one of claims 1 to 5 which when applied to a person's skin reduces the electrical impedance of the skin to less than 10,000 ohms/cm², wherein the pad is operated between 0.1 Hz to 200 Mhz.

7. The two-sided pad according to any one of claims 1 to 6 which when applied to a person's skin reduces the electrical impedance of the skin to less than 5,000 ohms/cm², wherein the pad is operated between 0.1 Hz to 200 Mhz.

8. The two-sided pad according to any one of claims 1 to 7 which when applied to a person's skin hydrates the skin for the period of time that a delivery, sensing or monitoring device is in contact with the skin.

9. The use of a two-sided pad (10) comprising a first side of the pad (14) comprising a cleansing formulation, a second side of the pad (16) comprising a hydrating formulation, and an inert, liquid impervious backing (12) separating the first and second sides, for cleansing and hydrating an area on the surface of a person's skin for applying a delivery, sensing or monitoring device to the said area.

10. The use of a pad according to claim 9 wherein the device is a monitor selected from the group consisting of blood glucose monitors, cardiac function monitors, electro-myograph monitors, and wrist watches.

11. The use of a pad according to claims 9 or 10, wherein the two-sided pad (10) reduces the electrical impedance of the skin to less than 10,000 ohms/cm², wherein the pad is operated between 0.1 Hz to 200 Mhz.

12. The use of a pad according to any of claims 9-11, wherein the two-sided pad (10) reduces the electrical impedance of the skin to less than 5,000 ohms/cm², wherein the pad is operated between 0.1 Hz to 200 Mhz.

13. The use of a pad according to any of claims 9-12, wherein the two-sided pad (10) hydrates the skin for the period of time that the device is in contact with the skin.

## Patentansprüche

1. Zweiseitiges Kissen (10), umfassend
eine erste Seite des Kissens (14), umfassend eine Reinigungsformulierung, eine zweite Seite des Kissens (16), umfassend eine Hydratisierungsformulierung, und eine inerte, flüssigkeitsundurchlässige Trägerschicht (12), die die ersten und zweiten Seiten trennt.

2. Zweiseitiges Kissen (10) gemäß Anspruch 1, wobei die erste Seite (14) ein leicht schmirgelartiges Material umfasst.

3. Zweiseitiges Kissen (10) gemäß Anspruch 2, wobei die zweite Seite (16) dasselbe Material wie die erste Seite umfasst.

4. Zweiseitiges Kissen (10) gemäß Anspruch 1, wobei die Reinigungsformulierung eine Lösung oder Suspension ist, umfassend einen Alkohol, Wasser, ein Konservierungsmittel oder einen Geruchsstoff oder eine Kombination daraus.

5. Zweiseitiges Kissen (10) gemäß Anspruch 1, wobei die Hydratisierungsformulierung eine Lösung, Emulsion oder Suspension ist, umfassend ein Hydrogel, eine Lotion, ein Öl, ein Anfeuchtungsmittel, Harnstoff oder eine Kombination daraus.

6. Zweiseitiges Kissen gemäß einem der Ansprüche 1 bis 5, das die elektrische Impedanz der Haut auf weniger als 10.000 Ω/cm² reduziert, wenn es auf die Haut einer Person aufgebracht wird, wobei das Kissen zwischen 0,1 Hz und 200 MHz betrieben wird.

7. Zweiseitiges Kissen gemäß einem der Ansprüche 1 bis 6, das die elektrische Impedanz der Haut auf weniger als 5.000 Ω/cm² reduziert, wenn es auf die Haut einer Person aufgebracht wird, wobei das Kissen zwischen 0,1 Hz und 200 MHz betrieben wird.

8. Zweiseitiges Kissen gemäß einem der Ansprüche 1 bis 7, das, wenn es auf die Haut einer Person aufgebracht wird, die Haut für eine Zeitspanne hydratisiert, solange eine Zufuhr-, Sensor- oder Überwachungsvorrichtung sich in Kontakt mit der Haut befindet.

9. Verwendung eines zweiseitigen Kissens (10), umfassend eine erste Seite des Kissens (14), umfassend eine Reinigungsformulierung, eine zweite Seite des Kissens (16), umfassend eine Hydratisierungsformulierung, und eine inerte, flüssigkeitsundurchlässige Trägerschicht (12), die die ersten und zweiten Seiten trennt, zur Reinigung und Hydratisierung eines Bereichs auf der Oberfläche der Haut einer Person zum Aufbringen einer Zufuhr-, Sensor- oder Überprüfungsvorrichtung auf den Bereich.

10. Verwendung eines Kissens gemäß Anspruch 9, wobei die Vorrichtung ein Monitor ist, gewählt aus der Gruppe bestehend aus Blutglucosemonitoren, Herzfunktionsmonitoren, Elektromyographmonitoren und Armbanduhren.

11. Verwendung eines Kissens gemäß den Ansprüche 9 oder 10, wobei das zweiseitige Kissen (10) die elektrische Impedanz der Haut auf weniger als 10.000 Ω/cm² reduziert, wobei das Kissen zwischen 0,1 Hz und 200 MHz betrieben wird.

12. Verwendung eines Kissens gemäß einem der Ansprüche 9 bis 11, wobei das zweiseitige Kissen (10) die elektrische Impedanz der Haut auf weniger als 5.000 Ω/cm² reduziert, wobei das Kissen zwischen 0,1 Hz und 200 MHz betrieben wird.

13. Verwendung eines Kissens gemäß einem der Ansprüche 9 bis 12, wobei das zweiseitige Kissen (10) die Haut für eine Zeitspanne hydratisiert, in der die Vorrichtung sich in Kontakt mit der Haut befindet.

## Revendications

1. Tampon à deux côtés (10) comprenant
un premier côté du tampon (14) comprenant une formulation de nettoyage, un deuxième côté du tampon (16) comprenant une formulation hydratante, et un support imperméable aux liquides, inerte (12) séparant les premier et deuxième côtés.

2. Tampon à deux côtés (10) selon la revendication 1, dans lequel le premier côté (14) comprend un matériau légèrement abrasif.

3. Tampon à deux côtés (10) selon la revendication 2, dans lequel le deuxième côté (16) comprend le même matériau que le premier côté.

4. Tampon à deux côtés (10) selon la revendication 1, dans lequel la formulation de nettoyage est une solution ou une suspension comprenant un alcool, de l'eau, un conservateur, ou un parfum, ou une combinaison de ceux-ci.

5. Tampon à deux côtés (10) selon la revendication 1, dans lequel la formulation hydratante est une solution, une émulsion ou une suspension, comprenant un hydrogel, une lotion, une huile, un hydratant, de l'urée ou une combinaison de ceux-ci.

6. Tampon à deux côtés selon l'une quelconque des revendications 1 à 5, qui une fois appliqué sur la peau d'une personne réduit l'impédance électrique de la peau à moins de 10 000 ohms/cm², dans lequel le tampon est alimenté entre 0,1 Hz et 200 MHz.

7. Tampon à deux côtés selon l'une quelconque des revendications 1 à 6, qui une fois appliqué sur la peau d'une personne réduit l'impédance électrique de la peau à moins de 5 000 ohms/cm², dans lequel le tampon est alimenté entre 0,1 Hz et 200 MHz.

8. Tampon à deux côtés selon l'une quelconque des revendications 1 à 7, qui une fois appliqué sur la peau d'une personne hydrate la peau sur la période de temps pendant laquelle un dispositif de délivrance, détection ou surveillance est au contact de la peau.

9. Utilisation d'un tampon à deux côtés (10) comprenant un premier côté du tampon (14) comprenant une formulation de nettoyage, un deuxième côté du tampon (16) comprenant une formulation hydratante, et un support imperméable aux liquides, inerte (12) séparant les premier et deuxième côtés, pour nettoyer et hydrater une zone à la surface de la peau d'une personne pour l'application d'un dispositif de délivrance, détection ou surveillance à ladite zone.

10. Utilisation d'un tampon selon la revendication 9, dans lequel le dispositif est un moniteur choisi dans le groupe constitué par les appareils de mesure de la glycémie, les appareils de mesure de la fonction cardiaque, les appareils d'électromyographie, et les montres-bracelets.

11. Utilisation d'un tampon selon la revendication 9 ou 10, dans lequel le tampon à deux côtés (10) réduit l'impédance électrique de la peau à moins de 10 000 ohms/cm², dans lequel le tampon est alimenté entre 0,1 Hz et 200 MHz.

12. Utilisation d'un tampon selon l'une quelconque des revendications 9 à 11, dans lequel le tampon à deux côtés (10) réduit l'impédance électrique de la peau à moins de 5 000 ohms/cm², dans lequel le tampon est alimenté entre 0,1 Hz et 200 MHz.

13. Utilisation d'un tampon selon l'une quelconque des revendications 9 à 12, dans lequel le tampon à deux côtés (10) hydrate la peau sur la période de temps perdant laquelle le dispositif est au contact de la peau.
